# EUROPEAN PATENT APPLICATION

(11) **EP 4 588 482 A1**
(43) Date of publication of application: **23.07.2025**
(21) Application number: 23865727.4
(22) Date of filing: 03.08.2023
(51) Int. Cl.: A61K 36/258, A61K 31/573, A61K 31/58, A61K 45/06, A61P 31/12, A23L 33/10, A23L 33/105, A23K 10/30, A23K 20/168, A23K 10/12

(54) **ANTIVIRAL COMPOSITION CONTAINING GINSENG EXTRACT AND STEROID COMPOUND AS ACTIVE INGREDIENTS**

(30) Priority: 13.09.2022 KR 20220114707
(71) Applicant: Ginseng Bypharm Co., Ltd., Wonju-si Gangwon-do 26506 (KR)
(72) Inventor: AN, Junmin, Wonju-si Gangwon-do 26506 (KR)
(74) Representative: Cabinet Chaillot
(86) International application number: PCT/KR2023/011426
(87) International publication number: WO 2024/058427

(57) **Abstract**

A composition comprising a steroid compound and ginseng extracts selected from the group consisting of ginseng extract, red ginseng extract and black ginseng extract as active ingredients provided by the present invention has a wide range of inhibitory activity against viruses, and thus, it is useful for effectively preventing, ameliorating or treating infections caused by viruses or diseases caused by infections.

## Description

### [Technical Field]

The present invention relates to a composition comprising, as active ingredients, a steroid compound and any one or more selected from the group consisting of ginseng (including dried ginseng and fresh ginseng) extract, red ginseng extract and black ginseng extract, which may be considered ginsengs with a wide range of antiviral activity against coronaviruses, influenza viruses, adenoviruses, and the like.

### [Background Art]

Virus means toxic substance in Latin and refers to a group of infectious pathogenic particles that pass through bacterial filter paper (0.22 µm). Viruses exist around us, and some pathogenic microorganisms such as bacteria and mold and viruses cause harm to the human body, such as causing disease and producing bad odors.

Accordingly, there is growing interest in hygiene management products that aim to improve the environment for these viruses and microorganisms. For example, various products are being developed that may prevent highly epidemic viral infections such as SARS, influenza virus, new flu and MERS coronavirus (MERS-CoV), including coronavirus infectious disease-19 (COVID-19).

The influenza virus kills 250,000 to 500,000 people worldwide every year, infects 10 to 40% of children, and causes serious global diseases that cause national economic losses.

After the 1918 Spanish flu pandemic, influenza pandemics such as the 1957 Asian flu and the 1968 Hong Kong flu appeared every few decades, and with the emergence of the 2019 H1N1 influenza virus (2009pdmA), 270,000 people were infected worldwide, and 3,200 or more people died. Even though pandemic measures have been put in place due to the possibility of a highly contagious new variant spreading in the Southern Hemisphere, social ramifications and concerns have not abated.

Influenza is an acute respiratory disease accompanied by chills, fever, myalgia and cough, and is usually transmitted from person to person through aerosols discharged into the air when an infected person coughs or sneezes. It may also be transmitted through bird droppings and is compared to the common cold, wherein the common cold is a respiratory disease caused by infections with adenovirus, rhinovirus, coxsackie virus, coronavirus, and the like, and is usually not accompanied by muscle pain or rapid high fever. Influenza infection is caused by influenza viruses belonging to the *Orthomyxoviridae* family, and since these viruses are RNA viruses, antigenic variation is relatively easy, and thus, repeated reinfection within the infected population is possible.

Influenza viruses that cause human infection are influenza A and B, and viral antigenic variation is determined by hemagglutinin (HA) and neuraminidase (NA) on the virus surface. Subtypes H1-H16 and N1-N9 are generated depending on the genetic combination of the virus surface proteins HA and NA, and among these, the subtype of human influenza A virus is determined by different combinations of three types, H1, H2, and H3, and two types, N1 and N2. The reason influenza B cannot cause a pandemic is because, unlike influenza A, it only infects humans and does not acquire genes from animal viruses.

Adenoviruses (*Adenoviridae*) are medium-sized viruses of 90 to 100 nm and have no envelope. They are icosahedral in shape and have DNA in the form of a double helix. Adenovirus is the cause of 5 to 10% of upper respiratory diseases in children, and adults may also be infected.

Unlike other viruses, whose clinical course is relatively better than bacterial pneumonia, it is known that adenovirus may show symptoms similar to severe bacterial pneumonia and may cause fatal results or leave aftereffects such as permanent lung damage.

Viruses belonging to the *Adenoviridae* family may infect various vertebrates, including humans, and since adenovirus was first isolated from human adenoids, it was given the name "adenovirus." The most common symptom of adenovirus infection is upper respiratory disease.

Adenovirus infection is often accompanied by conjunctivitis, tonsillitis, otitis media, laryngitis, gastroenteritis, and the like. In particular, children may suffer from bronchiolitis or pneumonia, and in rare cases, adenovirus may cause encephalitis or cystitis.

Coronavirus is an RNA virus that causes respiratory diseases, digestive diseases, liver diseases, brain diseases, and the like in mammals and birds (Gallagher TM. et.al., Virology, 279(2):371-374, 2001). In particular, among viruses belonging to the *Coronaviridae* family, porcine transmissible gastroenteritis virus (TGEV) and porcine epidemic diarrhea virus (PEDV) cause highly contagious viral diseases, and are viruses that invade the gastrointestinal digestive system to cause dehydration due to vomiting and diarrhea and high fever, and also have a high lethality to cause significant economic losses (Duarte M. et.al., J Gen Virol., 75 (Pt 5):1195-1200, 1994).

Although these viruses have a very high lethality, like diseases caused by other viral infections, no definitive therapeutic agents have been developed.

Coronavirus infectious disease-19 (coronavirus disease 2019, COVID-19) or corona-19 caused by a coronavirus is a respiratory infectious disease caused by a new type of coronavirus (SARSCoV-2; RNA virus belonging to *Coronaviridae*) that first emerged in Wuhan, China in December 2019 and has since spread throughout China and the world.

Initially, it was known only as a respiratory infectious disease of unknown cause, but the pathogen was confirmed, when the World Health Organization (WHO) announced on January 9, 2020 that the cause of the pneumonia was a new type of coronavirus (SARS-CoV-2, named by the International Committee on Taxonomy of Viruses on February 11).

The pathogen of coronavirus infectious disease-19 is "SARS-coronavirus-2 (SARS-CoV-2)." On February 11, 2020, the International Committee on Taxonomy of Viruses (ICTV) published a paper proposing the name SARS-CoV-2 for the pathogen of corona-19, and the committee said that it was emphasizing that this virus is similar to the SARS (severe acute respiratory syndrome) outbreak in 2003.

Korea obtained and analyzed the genetic sequence of the virus released by China through academia, and confirmed that it had the highest homology (89.1%) to a bat-derived similar coronavirus. Homology to the four types of human coronaviruses was low at 39% to 43%, and 50% homology to MERS and 77.5% homology to SARS were confirmed.

Coronavirus infectious disease-19 (coronavirus disease 2019, COVID-19) disease, which is currently a worldwide problem, is defined as a respiratory syndrome caused by a new coronavirus (severe acute respiratory syndrome coronavirus 2, SARS-CoV-2) infection, and the disease classification is a class 1 infectious disease and a statutory infectious disease under the new infectious disease syndrome, and the disease code is U07.1.

The pathogen is SARS-CoV-2, which is an RNA virus belonging to the *Coronaviridae* family, and the route of transmission is currently through droplets (salivary droplets) or contact, and transmission mainly occurs through droplets produced when coughing or sneezing, or by touching an object contaminated with the coronavirus and then touching the eyes, nose or mouth.

The global lethality due to coronavirus infection is about 3.4% on average (WHO, 3.5 standard), and the level of lethality by country and age is very different, with 6% of infected people worldwide currently registered as dead, and severe and death cases mainly occur in elderly patients, patients with weakened immune function and patients with underlying diseases.

Coronavirus is an RNA virus with a helical structure of a single positive strand, and is composed of a total of five structural proteins, of which the spike glycoprotein (S protein) is observed under an electron microscope as if it has a crown or halo (Schoeman and Fielding, 2019). Coronavirus is the largest of the existing RNA viruses at 27 to 34 Kb, and is divided into four groups: alpha, beta, delta and gamma, of which the beta group is highly contagious and virulent.

SARS-CoV-2 invades when the S protein present on its surface binds to the angiotensin converting enzyme 2 (ACE2) of the host cell, and has the characteristic of being observed as viral pneumonia because ACE2 is widely distributed in the oral and nasal mucosa, nasopharynx, lungs, stomach, small intestine, large intestine, and the like (Hamming et al., 2004).

The incubation period of the coronavirus is 1 to 14 days (average 4 to 7 days), and the diagnosis is made by isolating the virus from the specimen or detecting a specific gene in the specimen according to the testing standards for diagnosis as a diagnostic standard.

Under these circumstances, many efforts are being made to overcome the shortcomings of existing antiviral agents, and as one of the efforts, research on the antiviral efficacy of herbal medicine extracts and plant extracts is actively underway in Korea.

However, since the antiviral effect is mostly minimal when these extracts are applied, there is a need for a solution thereto.

### [Disclosure]

### [Technical Problem]

Therefore, the problem to be solved by the present invention is to solve the above-mentioned problems and provide a composition that has excellent and a wide range of antiviral activity.

### [Technical Solution]

In order to solve the above problem, the present invention provides a composition for preventing, alleviating or treating viral infections, comprising:
ginseng extracts selected from the group consisting of ginseng extract, red ginseng extract and black ginseng extract; and
a steroid compound,
as active ingredients.

In the composition according to the present invention as described above, the ginseng extracts are characterized in that they are extracted using water or ethanol as an extraction solvent.

In the composition according to the present invention as described above, the ginseng extracts are characterized in that they are treated with any one or more enzymes selected from the group consisting of β-galactosidase, β-glucosidase, β-glucanase, α-amylase and cellulase after the extraction.

In the composition according to the present invention as described above, the ginseng extracts are characterized in that they are fermented with lactic acid bacteria or yeast.

In the composition according to the present invention as described above, the ginseng extracts are characterized in that they are fermented with lactic acid bacteria or yeast after the enzyme treatment.

In the composition according to the present invention as described above, the steroid compound is characterized in that it is any one or more selected from the group consisting of dexamethasone, hydrocortisone, prednisolone, methylprednisolone and triamcinolone.

In the composition according to the present invention as described above, the virus may be any one of coronavirus, influenza virus and adenovirus.

### [Advantageous Effects]

The composition provided by the present invention may have a very remarkable inhibitory activity against a wide range of viruses such as coronavirus, influenza virus and adenovirus, thereby effectively preventing, ameliorating or treating infections caused by viruses or diseases caused by infections.

In particular, the use of steroid compounds may be disadvantageous in terms of inhibiting viruses, but even though the composition of the present invention comprises a steroid compound, it has the characteristics of being able to fully utilize the useful aspects of steroid compounds for people infected with viruses, such as solving the problems caused by the use of steroid compounds by remarkably increasing antiviral effects such as inhibition of virus proliferation, and at the same time having the effect of alleviating inflammation such as pneumonia or bronchitis.

Therefore, the composition according to the present invention is an excellent composition in both the anti-inflammatory effect that may alleviate symptoms caused by viral infection and the antiviral effect that may inhibit virus proliferation.

### [Best Mode]

Hereinafter, specific details for carrying out the present invention will be described in detail.

The present invention has been completed after confirming that it was effective as a composition that may prevent, ameliorate or treat viral infections because it showed broad and excellent antiviral activity and even had excellent anti-inflammatory effects of steroid compounds against inflammation such as pneumonia and bronchitis when the steroid compound was used together with ginseng extracts selected from the group consisting of ginseng extract, red ginseng extract and black ginseng extract, an enzyme-treated product of such ginseng extracts and a fermented product of the ginseng extracts, and thus, the present invention provides a composition for preventing, alleviating or treating viral infections, comprising ginseng extracts and the like and a steroid compound as active ingredients.

In the present invention as described above, the steroid compound refers to all fat-soluble compounds with a steroid nucleus such as sterols, bile acids and sex hormones, and is a lipid and an organic compound that does not contain fatty acids and has a common basic structure in which four rings, i.e., three rings of six carbon atoms and one ring of five carbon atoms are bonded, and is not particularly limited as long as it is widely known in the technical field to which the present invention pertains, but is preferably one or more selected from the group consisting of dexamethasone, hydrocortisone, prednisolone, methylprednisolone and triamcinolone.

These steroids have strong anti-inflammatory effects, thereby playing a key role in treating several conditions, including exacerbated asthma and lung disease, and are effective in alleviating symptoms such as bronchitis and pneumonia caused by viral infections, or preventing or treating their severity, and the like, but may cause the problem of delaying the elimination of viruses by weakening immunity; however, in the present invention, they are designed to be used together with ginseng extracts to further enhance the antiviral effect of inhibiting virus proliferation while maintaining the anti-inflammatory effect of the steroids.

In the present invention as described above, the steroid compound is currently approved as a drug and may be used according to the standard amount, but preferably, per 60 kg of the subject to be administered on a daily basis, dexamethasone is 1.5 to 10 mg, hydrocortisone is 10 to 120 mg, prednisolone or methylprednisolone is 10 to 60 mg, and triamcinolone is 8 to 16 mg.

The ginseng extracts used in the present invention collectively refer to ginseng extract (including dried ginseng extract and fresh ginseng extract), red ginseng extract and black ginseng extracts, and the preparation method of red ginseng and the preparation method of black ginseng may be used without particular limitation as long as they are widely known in the technical field to which the present invention pertains, that is, ginseng, red ginseng and black ginseng that are commonly used and distributed may be used without particular limitation.

In addition, the extraction method of the present invention may be used without particular limitation as long as it is widely known in the technical field to which the present invention pertains, and as an example thereof, it may be selected and used from methods such as heat extraction, cold maceration extraction, reflux cooling extraction, steam distillation, ultrasonic extraction, elutriation and expression, and depending on the purpose, the extract may be subjected to a conventional fractionation process and purified according to a conventional purification method.

In the present invention, extracts or mixtures thereof extracted by a conventional extraction method using water or ethanol as an extraction solvent are preferably used as the ginseng extracts.

In addition, they may be prepared by powdering the extracted first extract through additional processes such as reduced pressure distillation and freeze-drying or spray drying, and purified fractions may be obtained through various chromatographies such as silica gel column chromatography, high performance liquid chromatography and thin layer chromatography.

Therefore, the extract in the present invention may be understood as a concept that includes all extracts, separated compounds, fractions and purified products obtained at each stage of extraction, fractionation or purification, as well as dilutions, concentrates and dried products thereof.

In addition, in the present invention, the ginseng extracts may be used by being enzyme-treated or fermented, or may be used by being enzyme-treated and then fermented.

The enzymes used in the present invention are preferably any one or more selected from the group consisting of β-galactosidase, β-glucosidase, β-glucanase, α-amylase and cellulase.

The strain used for fermentation of the present invention may be all strain known to ferment ginseng extracts, and may preferably be lactic acid bacteria or yeast.

Specifically, the preparation of ginseng extracts used in the present invention may be accomplished through:
an extract step of extracting any one or more ginsengs selected from the group consisting of ginseng (dried ginseng or fresh ginseng), red ginseng and black ginseng with hot water at 70 to 100°C, or an extract step of extracting the ginsengs using 70% by weight of ethanol;
an enzyme treatment step of adding an enzyme to the extract in the extraction step and enzyme-treating it at 25 to 65°C for 2 to 7 hours; and
a fermentation step of adding lactic acid bacteria or yeast, preferably *Saccharomyces cerevisiae* to the enzyme-treated extract and fermenting it at 20 to 40°C for 10 to 48 hours.

The amount of ginseng extracts used as described above is not particularly limited, but is preferably 2 to 5 g per 60 kg of the subject to be administered on a daily solid basis.

The composition according to the present invention consisting of the black ginseng extract and the steroid compounds as described above may be administered once a day at the daily usage described above, but is preferably administered in two to three divided doses.

The composition according to the present invention as described above is expected to exhibit antiviral activity without particular limitations because it improves the mRNA expression level of Ifnb1 (interferon beta), which is a type 1 interferon showing a wide range of antiviral activity, and the mRNA expression levels of genes Mx1 and Mx2 that express Mx1 (Mx dynamin like GTPase 1) and Mx2 (Mx dynamin like GTPase 2), and shows antiviral activity against, for example, *Flaviviridae, Adenoviridae, Coronaviridae, Reoviridae, Picornaviridae, Caliciviridae, Togaviridae, Arenaviridae, Orthomyxoviridae, Paramyxoviridae, Bunyaviridae, Rhabdoviridae, Filoviridae, Astroviridae, Bornnaviridae* and *Arteriviridae,* preferably coronavirus, influenza virus and adenovirus.

In particular, in the case of the coronavirus, it shows very excellent antiviral activity against all of HCoV-229E, HCoV-NL63, SARS-CoV, MERS-CoV, HCoV-OC43, HCoV-HKU1 and SARS-CoV-2.

The composition of the present invention as described above may be used for pharmaceutical compositions, food compositions, food additive compositions, feed compositions or feed additive compositions, and its use with additives, excipients and other antiviral agents used to prepare these compositions is not particularly limited to ordinary procedures, and the formulation of the composition is also not particularly limited.

Hereinafter, the present invention will be described in more detail through examples and test examples. It will be apparent to those skilled in the art that these examples are only for illustrate the present invention in more detail and the scope of the present invention is not limited to these examples in accordance with the gist of the present invention.

### Examples using ginseng

### Preparation of composition

As the ginseng extract, a hot water extract, an enzyme-treated product treated with α-amylase after extraction, and a fermented product fermented with *Saccharomyces cerevisiae* after extraction were used.

Specifically, it was prepared through:
an extraction step of extracting fresh ginseng with hot water at 70 to 100°C; and
an enzyme treatment step of adding an enzyme to the extract in the extraction step and enzyme-treating it at 45°C for 3 hours; or
a fermentation step of adding *Saccharomyces cerevisiae* to the extract in the extraction step and fermenting it at 30°C for 24 hours.

A composition according to the present invention was prepared by combining each ginseng extract with any one selected from the group consisting of dexamethasone, hydrocortisone, prednisolone and triamcinolone, and compositions using ginseng extract and a steroid compound alone were used as comparative examples, and their composition is as shown in Table 1 below.

**[Table 1]**

| | Composition |
|---|---|
| Example 1 | Ginseng hot water extract + dexamethasone |
| Example 2 | Ginseng hot water extract + hydrocortisone |
| Example 3 | Ginseng hot water extract + prednisolone |
| Example 4 | Ginseng hot water extract + triamcinolone |
| Example 5 | Enzyme-treated product of ginseng hot water extract + dexamethasone |
| Example 6 | Enzyme-treated product of ginseng hot water extract + hydrocortisone |
| Example 7 | Enzyme-treated product of ginseng hot water extract + prednisolone |
| Example 8 | Enzyme-treated product of ginseng hot water extract + triamcinolone |
| Example 9 | Fermented product of ginseng hot water extract + dexamethasone |
| Example 10 | Fermented product of ginseng hot water extract + hydrocortisone |
| Example 11 | Fermented product of ginseng hot water extract + prednisolone |
| Example 12 | Fermented product of ginseng hot water extract + triamcinolone |
| Comparative Example 1 | Ginseng hot water extract |
| Comparative Example 2 | Enzyme-treated product of ginseng hot water extract |
| Comparative Example 3 | Fermented product of ginseng hot water extract |
| Comparative Example 4 | Dexamethasone |
| Comparative Example 5 | Hydrocortisone |
| Comparative Example 6 | Prednisolone |
| Comparative Example 7 | Triamcinolone |

### Test Example 1:

### Preparation of mice

The present inventors purchased 8-week-old C57BL/6 male mice from Central Lab. Animal Inc. and used them in the experiments below. The mice were acclimatized by providing sufficient feed and water for one week in a laboratory environment under the conditions of a temperature of 21 ± 2°C, humidity of 50 ± 10% and 12 hours/12 hours (dark/light cycle).

Subsequently, 8-week-old C57BL/6 mice were orally administered 22.5 mg/kg of ginseng extract in solid form in combination with 0.03 mg/kg of dexamethasone, 2.5 mg/kg of hydrocortisone, 0.1 mg/kg of prednisolone and 0.1 mg/kg of triamcinolone twice a day for 2 weeks, and then sacrificed, and the liver was enucleated and used in the experiment below.

### Confirmation of antiviral effect

To confirm the antiviral effect of the compositions according to the present invention prepared in the examples and the compositions of the comparative examples, livers enucleated from the group administered and the control group not administered the compositions to mice were pulverized with TissueLyser II (Qiagen), and then total RNA was isolated using the RNeasy mini kit (Qiagen). Afterwards, reverse transcription (RT) was performed with 2 µm of RNA using the RevertAid RT Kit (EP0441, Thermo Fisher Scientific), and real-time qRT-PCR was performed using the SensiFAST SYBR Hi-ROX kit (BIO92020, Bioline, London, UK).

The qPCR results are quantification of the target gene through Gapdh, which is a house-keeping gene, and are shown in Table 2 for the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, and the values in Table 2 refer to relative expression levels based on the expression level of the non-administered control group being set to 1.

**[Table 2]**

| | Relative mRNA expression level | | |
|---|---|---|---|
| | Ifnb1 | Mx1 | Mx2 |
| Example 1 | 3.0±0.5 | 4.4±0.5 | 5.7±0. 6 |
| Example 2 | 2.9±0.5 | 4.4±0.5 | 5.7±0. 5 |
| Example 3 | 3.0±0.5 | 4.5±0.5 | 5.7±0. 5 |
| Example 4 | 3.0±0.5 | 4.5±0.5 | 5.7±0. 5 |
| Example 5 | 3.5±0.5 | 5.1±0.5 | 6.4±0.6 |
| Example 6 | 3.5±0.5 | 5.1±0.5 | 6.3±0.6 |
| Example 7 | 3.5±0.5 | 5.1±0.5 | 6.4±0.6 |
| Example 8 | 3.6±0.5 | 5.0±0.5 | 6.4±0.6 |
| Example 9 | 3.5±0.5 | 5.2±0.5 | 6.4±0.6 |
| Example 10 | 3.6±0.5 | 5.1±0.5 | 6.5±0.6 |
| Example 11 | 3.5±0.5 | 5.1±0.5 | 6.4±0.6 |
| Example 12 | 3.5±0.5 | 5.1±0.5 | 6.4±0.6 |
| Comparative Example 1 | 1.7±0.2 | 2.2±0.2 | 2.7±0.3 |
| Comparative Example 2 | 1.8±0.3 | 2.4±0.3 | 2.7±0.2 |
| Comparative Example 3 | 1.9±0.3 | 2.4±0.3 | 2.9±0.3 |
| Comparative Example 4 | 0.7±0.2 | 0. 7±0. 2 | 0.8±0.3 |
| Comparative Example 5 | 0.7±0.2 | 0.6±0.2 | 0.7±0.3 |
| Comparative Example 6 | 0.8±0.2 | 0. 8±0. 2 | 0.8±0.3 |
| Comparative Example 7 | 0.7±0.2 | 0.9±0.2 | 0.7±0.3 |

Looking at Table 2 above, it could be confirmed that the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, were increased in both the compositions according to the present invention and the compositions of the comparative examples using ginseng extract alone, but the degree of increase was more remarkable in the compositions according to the present invention than in the compositions of the comparative examples.

In addition, since it is rather reduced in Comparative Examples 4 to 7 using steroid compounds, the use of steroid compounds may be disadvantageous in terms of inhibiting viruses, but even though the composition of the present invention comprises a steroid compound, it has the characteristics of being able to fully utilize the useful aspects for people infected with viruses, because it solves the problems caused by the use of steroid compounds by remarkably increasing antiviral effects, and at the same time has the effect of alleviating inflammation such as pneumonia or bronchitis.

In addition, when comparing the examples according to the present invention, the degree of increase in the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, was improved in the examples in which the ginseng extract was enzyme-treated or fermented.

In the above, type 1 interferons (type I interferons; IFNs) are proteins mainly secreted from virus-infected cells, include IFN-α and IFN-β as the most representative type 1 interferons in humans, and exhibit a wide range of antiviral effects (Nat Rev Immunol. 2015 Feb;15(2):87-103).

In addition, both Mx1 protein (interferon-induced GTP-binding protein Mx1) and Mx2 protein (interferon-induced GTP-binding protein MX2) are induced by type 1 interferons, and the Mx proteins induced by interferons correspond to markers known to accumulate in the cytoplasm in humans and bind to invading viruses to induce death of the viruses (Trends Microbiol. 2015 Mar;23(3):154-63, Microbiol Mol Biol Rev. 2013 Dec;77(4):551-66) .

Therefore, the above results mean that an antiviral effect appears when the compositions according to the present invention are administered in viral infections, and means that they may also be utilized to effectively prevent, ameliorate or treat viral infections or diseases caused by infections.

### Test Example 2:

### Confirmation of antiviral effect through in vitro experiments

Vero-E6 cells were cultured in DMEM(-/-), and inoculated into a 12 well-plate at a concentration of 5×10⁵ cells/well in the virus infection experiment. The cells were cultured in a CO₂ incubator at 37°C for 24 hours, and washed twice with 1×PBS after removing the cell medium.

Samples with a concentration of 100 ug/ml were pretreated with the composition of Test Example 1 above, and then cultured in a CO₂ incubator at 37°C for 2 hours, and subsequently, treated with the viruses as shown in Table 3 at a concentration of 50 pfu/well for 1 hour after removing the cell medium for virus infection. Afterwards, after the cell medium was removed, 1.5 ml of medium was added to each well and reacted for 72 hours, and then the results were confirmed and shown in Table 3, and the values in Table 3 are relative virus titers based on the virus titer of the non-treated control group being set to 100.

**[Table 3]**

| | Relative virus titer | | |
|---|---|---|---|
| | Influenza A virus | Adenovirus type 5 | SARS-CoV-2 virus |
| Example 1 | 28±1.3 | 28±1.3 | 29±1.3 |
| Example 2 | 28±1.3 | 28±1.3 | 28±1.3 |
| Example 3 | 29±1.3 | 28±1.3 | 28±1.3 |
| Example 4 | 28±1.3 | 29±1.3 | 29±1.3 |
| Example 5 | 23±1.3 | 22±1.3 | 22±1.3 |
| Example 6 | 22±1.3 | 23±1.3 | 23±1.3 |
| Example 7 | 23±1.3 | 23±1.3 | 22±1.3 |
| Example 8 | 22±1.3 | 23±1.3 | 22±1.3 |
| Example 9 | 22±1.3 | 23±1.3 | 22±1.3 |
| Example 10 | 22±1.3 | 23±1.3 | 23±1.3 |
| Example 11 | 23±1.3 | 22±1.3 | 22±1.3 |
| Example 12 | 23±1.3 | 22±1.3 | 22±1.3 |
| Comparative Example 1 | 54±1.9 | 56±1.9 | 59±1.9 |
| Comparative Example 2 | 52±1.9 | 54±1.9 | 57±1.9 |
| Comparative Example 3 | 52±1.9 | 54±1.9 | 57±1.9 |
| Comparative Example 4 | 102±1.9 | 102±1.9 | 101±1.9 |
| Comparative Example 5 | 102±1.9 | 103±1.9 | 102±1.9 |
| Comparative Example 6 | 103±1.9 | 102±1.9 | 101±1.9 |
| Comparative Example 7 | 103±1.9 | 102±1.9 | 101±1.9 |

As a result of the test in Table 3, viral RNA was reduced in both the compositions of the examples and the compositions of the comparative examples using ginseng extract alone, but it could be seen that when compared with the compositions of the comparative examples, the compositions according to the present invention showed reduced results of 70% or more, and thus, the antiviral activity was remarkably high.

In addition, since viral RNA shows increased results in Comparative Examples 4 to 7 using steroid compounds, they may be said to have an adverse effect in terms of inhibiting viral proliferation, but when used together with ginseng extracts as in the present invention, since the problems seen in Comparative Examples 4 to 7 are solved by increasing the antiviral activity of the ginseng extract, the composition of the present invention is a very useful composition that has strong antiviral activity and may fully utilize the anti-inflammatory effect of steroid compounds.

In addition, when comparing the examples according to the present invention, the degree of reduction in viral RNA was improved in the examples in which the ginseng extract was enzyme-treated or fermented.

### Test Example 3:

### Confirmation of antiviral effect through in vivo experiment

To confirm the antiviral effect *in vivo,* 9-week-old K18-ACE2 TG mice were used in the experiment. The mice were acclimatized by providing sufficient feed and water for one week in a laboratory environment under the conditions of a temperature of 21 ± 2°C, humidity of 50 ± 10% and 12 hours/12 hours (dark/light cycle), and were orally administered once, in the same manner as Test Example 1, starting one day before virus infection.

Three days after virus infection, each of the four orally administered mice was sacrificed to obtain lung tissue, and the virus titer in the lung tissue was measured through plaque assay and is shown in Table 4, and the values in Table 4 are relative virus titers based on the virus titer of the non-treated control group being set to 100.

**[Table 4]**

| | Relative virus titer | | |
|---|---|---|---|
| | Influenza A virus | Adenovirus type 5 | SARS-CoV-2 virus |
| Example 1 | 29±1.3 | 29±1.3 | 29±1.3 |
| Example 2 | 29±1.3 | 30±1.3 | 30±1.3 |
| Example 3 | 30±1.3 | 30±1.3 | 30±1.3 |
| Example 4 | 29±1.3 | 29±1.3 | 30±1.3 |
| Example 5 | 23±1.3 | 23±1.3 | 22±1.3 |
| Example 6 | 22±1.3 | 22±1.3 | 23±1.3 |
| Example 7 | 23±1.3 | 22±1.3 | 23±1.3 |
| Example 8 | 22±1.3 | 23±1.3 | 22±1.3 |
| Example 9 | 22±1.3 | 23±1.3 | 23±1.3 |
| Example 10 | 23±1.3 | 22±1.3 | 22±1.3 |
| Example 11 | 23±1.3 | 22±1.3 | 22±1.3 |
| Example 12 | 23±1.3 | 22±1.3 | 23±1.3 |
| Comparative Example 1 | 55±1.9 | 55±1.9 | 58±1.9 |
| Comparative Example 2 | 53±1.9 | 55±1.9 | 59±1.9 |
| Comparative Example 3 | 53±1.9 | 55±1.9 | 57±1.9 |
| Comparative Example 4 | 101±1.9 | 102±1.9 | 101±1.9 |
| Comparative Example 5 | 102±1.9 | 101±1.9 | 102±1.9 |
| Comparative Example 6 | 103±1.9 | 102±1.9 | 101±1.9 |
| Comparative Example 7 | 102±1.9 | 101±1.9 | 101±1.9 |

As a result of the test in Table 4, unlike the comparative examples using steroid compounds, the virus titer was reduced in both the compositions of the examples according to the present invention and the compositions of the comparative examples using ginseng extract, but it could be seen that when compared with the compositions of the comparative examples using ginseng extract, the compositions according to the present invention showed reduced results of 70% or more, and thus, the antiviral activity was remarkably high.

In addition, when comparing the examples according to the present invention, the degree of reduction in virus titer was improved in the examples in which the ginseng extract was enzyme-treated or fermented.

### Examples using red ginseng

### Preparation of composition

It is the same as the examples using ginseng except that red ginseng extract was used, and the comparative examples using steroid compounds alone are also omitted because they are the same as the test results in the examples using ginseng, and the composition of the examples and comparative examples is as shown in Table 5 below.

**[Table 5]**

| | Composition |
|---|---|
| Example 1 | Red ginseng hot water extract + dexamethasone |
| Example 2 | Red ginseng hot water extract + hydrocortisone |
| Example 3 | Red ginseng hot water extract + prednisolone |
| Example 4 | Red ginseng hot water extract + triamcinolone |
| Example 5 | Enzyme-treated product of red ginseng hot water extract + dexamethasone |
| Example 6 | Enzyme-treated product of red ginseng hot water extract + hydrocortisone |
| Example 7 | Enzyme-treated product of red ginseng hot water extract + prednisolone |
| Example 8 | Enzyme-treated product of red ginseng hot water extract + triamcinolone |
| Example 9 | Fermented product of red ginseng hot water extract + dexamethasone |
| Example 10 | Fermented product of red ginseng hot water extract + hydrocortisone |
| Example 11 | Fermented product of red ginseng hot water extract + prednisolone |
| Example 12 | Fermented product of red ginseng hot water extract + triamcinolone |
| Comparative Example 1 | Red ginseng hot water extract |
| Comparative Example 2 | Enzyme-treated product of red ginseng hot water extract |
| Comparative Example 3 | Fermented product of red ginseng hot water extract |

### Test Example 1:

### Preparation of mice

The present inventors purchased 8-week-old C57BL/6 male mice from Central Lab. Animal Inc. and used them in the experiments below. The mice were acclimatized by providing sufficient feed and water for one week in a laboratory environment under the conditions of a temperature of 21 ± 2°C, humidity of 50 ± 10% and 12 hours/12 hours (dark/light cycle).

Subsequently, 8-week-old C57BL/6 mice were orally administered 22.5 mg/kg of red ginseng extract in solid form in combination with 0.03 mg/kg of dexamethasone, 2.5 mg/kg of hydrocortisone, 0.1 mg/kg of prednisolone and 0.1 mg/kg of triamcinolone twice a day for 2 weeks, and then sacrificed, and the liver was enucleated and used in the experiment below.

### Confirmation of antiviral effect

To confirm the antiviral effect of the compositions according to the present invention prepared in the examples and the compositions of the comparative examples, livers enucleated from the group administered and the control group not administered the compositions to mice were pulverized with TissueLyser II (Qiagen), and then total RNA was isolated using the RNeasy mini kit (Qiagen). Afterwards, reverse transcription (RT) was performed with 2 µm of RNA using the RevertAid RT Kit (EP0441, Thermo Fisher Scientific), and real-time qRT-PCR was performed using the SensiFAST SYBR Hi-ROX kit (BIO92020, Bioline, London, UK).

The qPCR results are quantification of the target gene through Gapdh, which is a house-keeping gene, and are shown in Table 6 for the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, and the values in Table 6 refer to relative expression levels based on the expression level of the non-administered control group being set to 1.

**[Table 6]**

| | Relative mRNA expression level | | |
|---|---|---|---|
| | Ifnb1 | Mx1 | Mx2 |
| Example 1 | 3.5±0.5 | 5.1±0.5 | 6.1±0.6 |
| Example 2 | 3.5±0.5 | 5.2±0.5 | 6.2±0.5 |
| Example 3 | 3.6±0.5 | 5.1±0.5 | 6.2±0.5 |
| Example 4 | 3.5±0.5 | 5.2±0.5 | 6.1±0.5 |
| Example 5 | 4.8±0.5 | 6.2±0.5 | 6.7±0.6 |
| Example 6 | 4.7±0.5 | 6.2±0.5 | 6.8±0.6 |
| Example 7 | 4.8±0.5 | 6.1±0.5 | 6.9±0.6 |
| Example 8 | 4.7±0.5 | 6.3±0.5 | 6.8±0.6 |
| Example 9 | 4.7±0.5 | 6.2±0.5 | 6.8±0.6 |
| Example 10 | 4.8±0.5 | 6.1±0.5 | 6.8±0.6 |
| Example 11 | 4.7±0.5 | 6.3±0.5 | 6.8±0.6 |
| Example 12 | 4.8±0.5 | 6.2±0.5 | 6.9±0.6 |
| Comparative Example 1 | 2.4±0.2 | 2.7±0.2 | 3.2±0.3 |
| Comparative Example 2 | 2.4±0.3 | 2.6±0.3 | 3.1±0.2 |
| Comparative Example 3 | 2.3±0.3 | 2.7±0.3 | 3.1±0.3 |

Looking at Table 6 above, it could be confirmed that the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, were increased in both the compositions according to the present invention and the compositions of the comparative examples using red ginseng extract alone, but the degree of increase was more remarkable in the compositions according to the present invention than in the compositions of the comparative examples, and when comparing the examples according to the present invention, the degree of increase was further improved in the examples in which the red ginseng extract was enzyme-treated or fermented.

In addition, when compared to the examples using ginseng, the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, were overall increased in this example using red ginseng, meaning that red ginseng is superior to ginseng in terms of antiviral activity.

In the above, type 1 interferons (type I interferons; IFNs) are proteins mainly secreted from virus-infected cells, include IFN-α and IFN-β as the most representative type 1 interferons in humans, and exhibit a wide range of antiviral effects (Nat Rev Immunol. 2015 Feb;15(2):87-103).

In addition, both Mx1 protein (interferon-induced GTP-binding protein Mx1) and Mx2 protein (interferon-induced GTP-binding protein MX2) are induced by type 1 interferons, and the Mx proteins induced by interferons correspond to markers known to accumulate in the cytoplasm in humans and bind to invading viruses to induce death of the viruses (Trends Microbiol. 2015 Mar;23(3):154-63, Microbiol Mol Biol Rev. 2013 December;77(4):551-66).

Therefore, the above results mean that an antiviral effect appears when the compositions according to the present invention are administered in viral infections, and means that they may also be utilized to effectively prevent, ameliorate or treat viral infections or diseases caused by infections.

### Test Example 2:

### Confirmation of antiviral effect through in vitro experiment

Vero-E6 cells were cultured in DMEM(-/-), and inoculated into a 12 well-plate at a concentration of 5×10⁵ cells/well in the virus infection experiment. The cells were cultured in a CO₂ incubator at 37°C for 24 hours, and washed twice with 1×PBS after removing the cell medium.

Samples with a concentration of 100 ug/ml were pretreated with the composition of Test Example 1 above, and then cultured in a CO₂ incubator at 37°C for 2 hours, and subsequently, treated with the viruses as shown in Table 3 at a concentration of 50 pfu/well for 1 hour after removing the cell medium for virus infection. Afterwards, after the cell medium was removed, 1.5 ml of medium was added to each well and reacted for 72 hours, and then the results were confirmed and shown in Table 7, and the values in Table 7 are relative virus titers based on the virus titer of the non-treated control group being set to 100.

**[Table 7]**

| | Relative virus titer | | |
|---|---|---|---|
| | Influenza A virus | Adenovirus type 5 | SARS-CoV-2 virus |
| Example 1 | 24±1.3 | 23±1.3 | 24±1.3 |
| Example 2 | 24±1.3 | 24±1.3 | 23±1.3 |
| Example 3 | 23±1.3 | 22±1.3 | 23±1.3 |
| Example 4 | 24±1.3 | 24±1.3 | 23±1.3 |
| Example 5 | 19±1.3 | 18±1.3 | 18±1.3 |
| Example 6 | 19±1.3 | 19±1.3 | 19±1.3 |
| Example 7 | 19±1.3 | 19±1.3 | 19±1.3 |
| Example 8 | 19±1.3 | 19±1.3 | 18±1.3 |
| Example 9 | 18±1.3 | 18±1.3 | 19±1.3 |
| Example 10 | 19±1.3 | 19±1.3 | 18±1.3 |
| Example 11 | 19±1.3 | 19±1.3 | 19±1.3 |
| Example 12 | 19±1.3 | 19±1.3 | 19±1.3 |
| Comparative Example 1 | 50±1.9 | 51±1.9 | 54±1.9 |
| Comparative Example 2 | 48±1.9 | 49±1.9 | 52±1.9 |
| Comparative Example 3 | 47±1.9 | 49±1.9 | 51±1.9 |

As a result of the test in Table 7, viral RNA was reduced in both the compositions of the examples and the compositions of the comparative examples using red ginseng extract alone, but it could be seen that when compared with the compositions of the comparative examples, the compositions according to the present invention showed reduced results of 70% or more, and thus, the antiviral activity was remarkably high, and when comparing the examples according to the present invention, the degree of reduction in viral RNA was improved in the examples in which the red ginseng extract was enzyme-treated or fermented.

In addition, when compared to the examples using ginseng, the reduction of viral RNA was overall improved in this example using red ginseng, meaning that red ginseng is superior to ginseng in terms of antiviral activity.

### Test Example 3:

### Confirmation of antiviral effect through in vivo experiment

To confirm the antiviral effect *in vivo,* 9-week-old K18-ACE2 TG mice were used in the experiment. The mice were acclimatized by providing sufficient feed and water for one week in a laboratory environment under the conditions of a temperature of 21 ± 2°C, humidity of 50 ± 10% and 12 hours/12 hours (dark/light cycle), and were orally administered once, in the same manner as Test Example 1, starting one day before virus infection.

Three days after virus infection, each of the four orally administered mice was sacrificed to obtain lung tissue, and the virus titer in the lung tissue was measured through plaque assay and is shown in Table 8, and the values in Table 8 are relative virus titers based on the virus titer of the non-treated control group being set to 100.

**[Table 8]**

| | Relative virus titer | | |
|---|---|---|---|
| | Influenza A virus | Adenovirus type 5 | SARS-CoV-2 virus |
| Example 1 | 24±1.3 | 23±1.3 | 24±1.3 |
| Example 2 | 25±1.3 | 24±1.3 | 26±1.3 |
| Example 3 | 24±1.3 | 24±1.3 | 26±1.3 |
| Example 4 | 24±1.3 | 24±1.3 | 26±1.3 |
| Example 5 | 17±1.3 | 19±1.3 | 17±1.3 |
| Example 6 | 18±1.3 | 17±1.3 | 18±1.3 |
| Example 7 | 19±1.3 | 18±1.3 | 17±1.3 |
| Example 8 | 18±1.3 | 18±1.3 | 17±1.3 |
| Example 9 | 18±1.3 | 17±1.3 | 18±1.3 |
| Example 10 | 19±1.3 | 19±1.3 | 18±1.3 |
| Example 11 | 19±1.3 | 19±1.3 | 17±1.3 |
| Example 12 | 18±1.3 | 19±1.3 | 18±1.3 |
| Comparative Example 1 | 47±1.9 | 49±1.9 | 49±1.9 |
| Comparative Example 2 | 45±1.9 | 47±1.9 | 46±1.9 |
| Comparative Example 3 | 45±1.9 | 48±1.9 | 46±1.9 |

As a result of the test in Table 8, the virus titer was reduced in both the compositions of the examples according to the present invention and the compositions of the comparative examples using red ginseng extract, but when compared with the compositions of the comparative examples using red ginseng extract, the degree of reduction showed remarkably improved results in the compositions according to the present invention, and when comparing the examples according to the present invention, the degree of reduction in virus titer was improved in the examples in which the red ginseng extract was enzyme-treated or fermented.

In addition, when compared to the examples using ginseng, the virus reduction was overall improved in this example using red ginseng, meaning that red ginseng is superior to ginseng in terms of antiviral activity.

### Examples using black ginseng

### Preparation of composition

### It is the same as the examples using ginseng except that black ginseng extract was used, and the comparative examples using steroid compounds alone are also omitted because they are the same as the test results in the examples using ginseng, and the composition of the examples and comparative examples is as shown in Table 9 below.

**[Table 9]**

| | Composition |
|---|---|
| Example 1 | Black ginseng hot water extract + dexamethasone |
| Example 2 | Black ginseng hot water extract + hydrocortisone |
| Example 3 | Black ginseng hot water extract + prednisolone |
| Example 4 | Black ginseng hot water extract + triamcinolone |
| Example 5 | Enzyme-treated product of black ginseng hot water extract + dexamethasone |
| Example 6 | Enzyme-treated product of black ginseng hot water extract + hydrocortisone |
| Example 7 | Enzyme-treated product of black ginseng hot water extract + prednisolone |
| Example 8 | Enzyme-treated product of black ginseng hot water extract + triamcinolone |
| Example 9 | Fermented product of black ginseng hot water extract + dexamethasone |
| Example 10 | Fermented product of black ginseng hot water extract + hydrocortisone |
| Example 11 | Fermented product of black ginseng hot water extract + prednisolone |
| Example 12 | Fermented product of black ginseng hot water extract + triamcinolone |
| Comparative Example 1 | Black ginseng hot water extract |
| Comparative Example 2 | Enzyme-treated product of black ginseng hot water extract |
| Comparative Example 3 | Fermented product of black ginseng hot water extract |

### Test Example 1:

### Preparation of mice

The present inventors purchased 8-week-old C57BL/6 male mice from Central Lab. Animal Inc. and used them in the experiments below. The mice were acclimatized by providing sufficient feed and water for one week in a laboratory environment under the conditions of a temperature of 21 ± 2°C, humidity of 50 ± 10% and 12 hours/12 hours (dark/light cycle).

Subsequently, 8-week-old C57BL/6 mice were orally administered 22.5 mg/kg of black ginseng extract in solid form in combination with 0.03 mg/kg of dexamethasone, 2.5 mg/kg of hydrocortisone, 0.1 mg/kg of prednisolone and 0.1 mg/kg of triamcinolone twice a day for 2 weeks, and then sacrificed, and the liver was enucleated and used in the experiment below.

### Confirmation of antiviral effect

To confirm the antiviral effect of the compositions according to the present invention prepared in the examples and the compositions of the comparative examples, livers enucleated from the group administered and the control group not administered the compositions to mice were pulverized with TissueLyser II (Qiagen), and then total RNA was isolated using the RNeasy mini kit (Qiagen). Afterwards, reverse transcription (RT) was performed with 2 µm of RNA using the RevertAid RT Kit (EP0441, Thermo Fisher Scientific), and real-time qRT-PCR was performed using the SensiFAST SYBR Hi-ROX kit (BIO92020, Bioline, London, UK).

The qPCR results are quantification of the target gene through Gapdh, which is a house-keeping gene, and are shown in Table 10 for the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, and the values in Table 10 refer to relative expression levels based on the expression level of the non-administered control group being set to 1.

**[Table 10]**

| | Relative mRNA expression level | | |
|---|---|---|---|
| | Ifnb1 | Mx1 | Mx2 |
| Example 1 | 4.1±0.5 | 5.7±0.5 | 6.6±0.6 |
| Example 2 | 4.2±0.5 | 5.8±0.5 | 6.6±0.5 |
| Example 3 | 4.1±0.5 | 5.8±0.5 | 6.5±0.5 |
| Example 4 | 4.1±0.5 | 5.7±0.5 | 6.6±0.5 |
| Example 5 | 5.4±0.5 | 6.9±0.5 | 7.3±0.6 |
| Example 6 | 5.4±0.5 | 7.0±0.5 | 7.3±0.6 |
| Example 7 | 5.5±0.5 | 7.0±0.5 | 7.3±0.6 |
| Example 8 | 5.5±0.5 | 6.9±0.5 | 7.2±0.6 |
| Example 9 | 5.4±0.5 | 7.0±0.5 | 7.4±0.6 |
| Example 10 | 5.5±0.5 | 7.0±0.5 | 7.3±0.6 |
| Example 11 | 5.5±0.5 | 7.0±0.5 | 7.3±0.6 |
| Example 12 | 5.4±0.5 | 7.0±0.5 | 7.3±0.6 |
| Comparative Example 1 | 2.9±0.2 | 3.1±0.2 | 3.6±0.3 |
| Comparative Example 2 | 3.1±0.3 | 3.4±0.3 | 3.9±0.2 |
| Comparative Example 3 | 3.2±0.3 | 3.4±0.3 | 3.8±0.3 |

Looking at Table 10 above, it could be confirmed that the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, were increased in both the compositions according to the present invention and the compositions of the comparative examples using black ginseng extract alone, but the degree of increase was more remarkable in the compositions according to the present invention than in the compositions of the comparative examples, and when comparing the examples according to the present invention, the degree of increase was further improved in the examples in which the black ginseng extract was enzyme-treated or fermented.

In addition, when compared with the examples using ginseng and the examples using red ginseng, the mRNA expression level of Ifnb1 (interferon beta), which is a type 1 interferon, and the mRNA expression levels of genes Mx1 and Mx2 that express Mx1 (Mx dynamin like GTPase 1) and Mx2 (Mx dynamin like GTPase 2), which are proteins exhibiting a wide range of antiviral effects, were overall increased in this example using black ginseng, meaning that black ginseng is superior to ginseng and red ginseng in terms of antiviral activity.

In the above, type 1 interferons (type I interferons; IFNs) are proteins mainly secreted from virus-infected cells, include IFN-α and IFN-β as the most representative type 1 interferons in humans, and exhibit a wide range of antiviral effects (Nat Rev Immunol. 2015 Feb;15(2):87-103).

In addition, both Mx1 protein (interferon-induced GTP-binding protein Mx1) and Mx2 protein (interferon-induced GTP-binding protein MX2) are induced by type 1 interferons, and the Mx proteins induced by interferons correspond to markers known to accumulate in the cytoplasm in humans and bind to invading viruses to induce death of the viruses (Trends Microbiol. 2015 Mar;23(3):154-63, Microbiol Mol Biol Rev. 2013 Dec;77(4):551-66).

Therefore, the above results mean that an antiviral effect appears when the compositions according to the present invention are administered in viral infections, and means that they may also be utilized to effectively prevent, ameliorate or treat viral infections or diseases caused by infections.

### Test Example 2:

### Confirmation of antiviral effect through in vitro experiment

Vero-E6 cells were cultured in DMEM(-/-), and inoculated into a 12 well-plate at a concentration of 5×10⁵ cells/well in the virus infection experiment. The cells were cultured in a CO₂ incubator at 37°C for 24 hours, and washed twice with 1×PBS after removing the cell medium.

Samples with a concentration of 100 ug/ml were pretreated with the composition of Test Example 1 above, and then cultured in a CO₂ incubator at 37°C for 2 hours, and subsequently, treated with the viruses as shown in Table 3 at a concentration of 50 pfu/well for 1 hour after removing the cell medium for virus infection. Afterwards, after the cell medium was removed, 1.5 ml of medium was added to each well and reacted for 72 hours, and then the results were confirmed and shown in Table 11, and the values in Table 11 are relative virus titers based on the virus titer of the non-treated control group being set to 100.

**[Table 11]**

| | Relative virus titer | | |
|---|---|---|---|
| | Influenza A virus | Adenovirus type 5 | SARS-CoV-2 virus |
| Example 1 | 20±1.3 | 19±1.3 | 20±1.3 |
| Example 2 | 19±1.3 | 20±1.3 | 19±1.3 |
| Example 3 | 20±1.3 | 21±1.3 | 19±1.3 |
| Example 4 | 20±1.3 | 21±1.3 | 19±1.3 |
| Example 5 | 12±1.3 | 12±1.3 | 10±1.3 |
| Example 6 | 11±1.3 | 12±1.3 | 10±1.3 |
| Example 7 | 11±1.3 | 12±1.3 | 11±1.3 |
| Example 8 | 12±1.3 | 10±1.3 | 10±1.3 |
| Example 9 | 11±1.3 | 11±1.3 | 10±1.3 |
| Example 10 | 11±1.3 | 11±1.3 | 11±1.3 |
| Example 11 | 11±1.3 | 12±1.3 | 11±1.3 |
| Example 12 | 12±1.3 | 11±1.3 | 11±1.3 |
| Comparative Example 1 | 42±1.9 | 41±1.9 | 44±1.9 |
| Comparative Example 2 | 40±1.9 | 39±1.9 | 41±1.9 |
| Comparative Example 3 | 40±1.9 | 39±1.9 | 41±1.9 |

As a result of the test in Table 11, viral RNA was reduced in both the compositions of the examples and the compositions of the comparative examples using black ginseng extract alone, but it could be seen that when compared with the compositions of the comparative examples, the compositions according to the present invention had a remarkably high antiviral activity, and when comparing the examples according to the present invention, the degree of reduction in viral RNA was improved in the examples in which the black ginseng extract was enzyme-treated or fermented.

In addition, when compared to the examples using ginseng and the examples using red ginseng, the reduction of viral RNA was overall improved in this example using black ginseng, meaning that black ginseng is superior to ginseng and red ginseng in terms of antiviral activity.

### Test Example 3:

### Confirmation of antiviral effect through in vivo experiment

To confirm the antiviral effect *in vivo,* 9-week-old K18-ACE2 TG mice were used in the experiment. The mice were acclimatized by providing sufficient feed and water for one week in a laboratory environment under the conditions of a temperature of 21 ± 2°C, humidity of 50 ± 10% and 12 hours/12 hours (dark/light cycle), and were orally administered once, in the same manner as Test Example 1, starting one day before virus infection.

Three days after virus infection, each of the four orally administered mice was sacrificed to obtain lung tissue, and the virus titer in the lung tissue was measured through plaque assay and is shown in Table 12, and the values in Table 12 are relative virus titers based on the virus titer of the non-treated control group being set to 100.

**[Table 12]**

| | Relative virus titer | | |
|---|---|---|---|
| | Influenza A virus | Adenovirus type 5 | SARS-CoV-2 virus |
| Example 1 | 19±1.3 | 17±1.3 | 19±1.3 |
| Example 2 | 20±1.3 | 19±1.3 | 20±1.3 |
| Example 3 | 19±1.3 | 18±1.3 | 20±1.3 |
| Example 4 | 19±1.3 | 19±1.3 | 19±1.3 |
| Example 5 | 11±1.3 | 10±1.3 | 10±1.3 |
| Example 6 | 11±1.3 | 11±1.3 | 11±1.3 |
| Example 7 | 10±1.3 | 11±1.3 | 10±1.3 |
| Example 8 | 11±1.3 | 10±1.3 | 11±1.3 |
| Example 9 | 10±1.3 | 11±1.3 | 10±1.3 |
| Example 10 | 10±1.3 | 11±1.3 | 11±1.3 |
| Example 11 | 11±1.3 | 11±1.3 | 11±1.3 |
| Example 12 | 18±1.3 | 10±1.3 | 10±1.3 |
| Comparative Example 1 | 41±1.9 | 41±1.9 | 42±1.9 |
| Comparative Example 2 | 39±1.9 | 39±1.9 | 40±1.9 |
| Comparative Example 3 | 39±1.9 | 39±1.9 | 39±1.9 |

As a result of the test in Table 12, the virus titer was reduced in both the compositions of the examples according to the present invention and the compositions of the comparative examples using black ginseng extract, but when compared with the compositions of the comparative examples using black ginseng extract, the degree of reduction showed remarkably improved results in the compositions according to the present invention, and when comparing the examples according to the present invention, the degree of reduction in virus titer was improved in the examples in which the black ginseng extract was enzyme-treated or fermented.

In addition, when compared to the examples using ginseng and the examples using red ginseng, the virus reduction was overall improved in this example using black ginseng, meaning that black ginseng is superior to ginseng and red ginseng in terms of antiviral activity.

Taking the above together, it could be seen that the compositions according to the present invention may be utilized as a therapeutic agent for infections or infectious diseases caused by various viruses.

Therefore, the composition provided by the present invention may have a very remarkable inhibitory activity against a wide range of viruses such as coronavirus, influenza virus and adenovirus, thereby effectively preventing, ameliorating or treating infections caused by viruses or diseases caused by infections.

In addition, the present invention has the advantage of eliminating side effects such as weakened immunity that may occur due to steroid compounds by using a steroid compound and ginseng extracts together.

Therefore, the composition according to the present invention is an excellent composition in both the anti-inflammatory effect that may alleviate symptoms caused by viral infection and the antiviral effect that may inhibit virus proliferation.

## Claims

1. A pharmaceutical composition for preventing, alleviating or treating viral infections, comprising a steroid compound and ginseng extracts selected from the group consisting of ginseng extract, red ginseng extract and black ginseng extract as active ingredients.

2. A health food composition for preventing or ameliorating viral infections, comprising a steroid compound and ginseng extracts selected from the group consisting of ginseng extract, red ginseng extract and black ginseng extract as active ingredients.

3. A feed composition for preventing or ameliorating viral infections, comprising a steroid compound and ginseng extracts selected from the group consisting of ginseng extract, red ginseng extract and black ginseng extract as active ingredients.

4. The composition according to any one of claims 1 to 3, which is **characterized in that** the virus is any one of coronavirus, influenza virus and adenovirus.

5. The composition according to any one of claims 1 to 3, which is **characterized in that** the ginseng extracts are extracted using water or ethanol as an extraction solvent.

6. The composition according to claim 5, which is **characterized in that** the ginseng extracts are treated with any one or more enzymes selected from the group consisting of β-galactosidase, β-glucosidase, β-glucanase, α-amylase and cellulase after the extraction.

7. The composition according to claim 5, which is **characterized in that** the ginseng extracts are fermented with lactic acid bacteria or yeast after the extraction.

8. The composition according to claim 6, which is **characterized in that** the ginseng extracts are fermented with lactic acid bacteria or yeast after the enzyme treatment.

9. The composition according to any one of claims 1 to 3, which is **characterized in that** the steroid compound is any one or more selected from the group consisting of dexamethasone, hydrocortisone, prednisolone, methylprednisolone and triamcinolone.
